# EUROPEAN PATENT APPLICATION

(11) **EP 3 392 303 A1**
(43) Date of publication of application: **24.10.2018**
(21) Application number: 16875450.5
(22) Date of filing: 05.12.2016
(51) Int. Cl.: C08L 21/00, B60C 1/00, B60C 5/14, C07C 243/38, C08K 3/04, C08K 5/25

(54) **RUBBER ADDITIVE, RUBBER COMPOSITION AND PNEUMATIC TIRE USING SAME**

(30) Priority: 14.12.2015 JP 2015243617
(71) Applicant: Bridgestone Corporation, Tokyo 104-8340 (JP); Otsuka Chemical Co., Ltd., Osaka-shi, Osaka 540-0021 (JP)
(72) Inventor: YOSHIZAWA Takahiro, Tokyo 104-8340 (JP); NAKASHIMA Shinya, Tokushima-shi Tokushima 771-0193 (JP); ABE Masaki, Tokushima-shi Tokushima 771-0193 (JP); KODAMA Kazuhiro, Tokushima-shi Tokushima 771-0193 (JP)
(74) Representative: Oxley, Robin John George
(86) International application number: PCT/JP2016/086071
(87) International publication number: WO 2017/104467

(57) **Abstract**

Provided are: a rubber additive that enables to provide a rubber composition having excellent low heat generation properties and manufacturing operability; a rubber composition including the rubber additive; and a pneumatic tire including the rubber composition. The rubber additive is characterized by containing a hydrazide compound represented by Formula (I) below, where R represents a hydroxyl group, an alkyl group having 1 to 4 carbon atoms, or an alkoxy group having 1 to 4 carbon atoms and A represents an alkylene group having 1 to 4 carbon atoms or an alkenylene group having 2 to 4 carbon atoms; the rubber composition containing the rubber additive; and the pneumatic tire including the rubber composition:

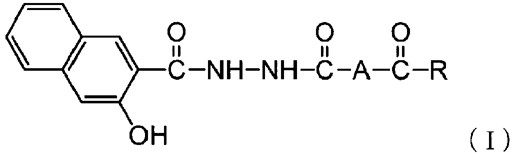

## Description

### TECHNICAL FIELD

The present invention relates to a rubber additive, a rubber composition, and a pneumatic tire comprising the same. More particularly, the present invention relates to: a rubber additive that enables to provide a rubber composition having excellent low heat generation properties and manufacturing operability; a rubber composition; and a pneumatic tire comprising the rubber composition.

### BACKGROUND ART

In pneumatic tires, for the purpose of reducing the rolling resistance and thereby achieving a reduction in fuel consumption, improvement in the low heat generation properties of rubber compositions used in pneumatic tires, that is, reduction in tan δ, has been studied for many years. However, incorporation of a specific compound into a rubber composition for an improvement in the low heat generation properties has problems in that, for example, it increases the pre-vulcanization viscosity of the rubber composition, impairing the manufacturing operability.

In order to solve such problems, for example, Patent Document 1 proposes to incorporate a specific compound having a hydrazide group into a rubber composition so as to improve the manufacturing operability by inhibiting an increase in viscosity while maintaining the low heat generation properties.

### RELATED ART DOCUMENT

### PATENT DOCUMENT

[Patent Document 1] Japanese Unexamined Patent Application Publication No. H10-330549

### SUMMARY OF THE INVENTION

### PROBLEMS TO BE SOLVED BY THE INVENTION

The tread rubber composition disclosed in Patent Document 1 made it possible to inhibit an increase in viscosity while maintaining the low heat generation properties; however, since this causes a reduction in scorch performance, there is still room for improvement in terms of manufacturing operability such as moldability.

In view of the above, an object of the present invention is to provide: a rubber additive that enables to provide a rubber composition having excellent low heat generation properties and manufacturing operability; a rubber composition comprising the rubber additive; and a pneumatic tire comprising the same.

### MEANS FOR SOLVING THE PROBLEMS

The present inventors intensively studied to solve the above-described problems and consequently discovered that the above-described object can be achieved by incorporating a prescribed amount of a specific compound having a hydrazide group as a rubber additive along with a prescribed amount of a filler into a rubber composition, thereby completing the present invention.

That is, the rubber additive of the present invention is characterized by comprising a hydrazide compound represented by the following Formula (I): 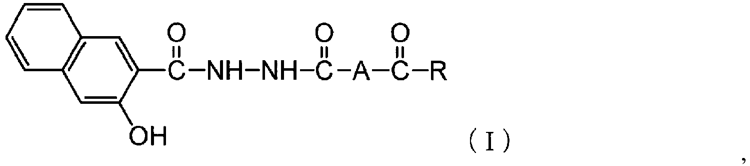 where R represents a hydroxyl group, an alkyl group having 1 to 4 carbon atoms, or an alkoxy group having 1 to 4 carbon atoms; and A represents an alkylene group having 1 to 4 carbon atoms or an alkenylene group having 2 to 4 carbon atoms.

Further, the rubber composition of the present invention is characterized by comprising, with respect to 100 parts by mass of a diene-based rubber component: 10 to 160 parts by mass of a filler; and 0.01 to 30 parts by mass of the above-described rubber additive of the present invention.

Still further, the pneumatic tire of the present invention is characterized by comprising the above-described rubber composition of the present invention.

Yet still further, the hydrazide compound of the present invention is characterized by being represented by the following Formula (I): 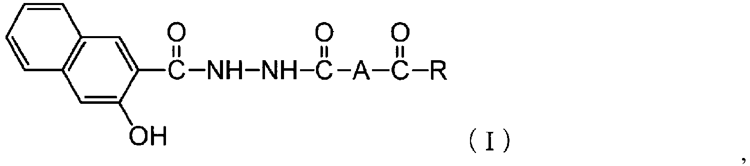 where R represents an alkoxy group having 1 to 4 carbon atoms; and A represents an alkenylene group having 2 to 4 carbon atoms.

### EFFECTS OF THE INVENTION

According to the present invention, a rubber additive that enables to provide a rubber composition having excellent low heat generation properties and manufacturing operability can be provided. In addition, according to the present invention, a rubber composition having excellent low heat generation properties and manufacturing operability as well as a pneumatic tire comprising the same can be provided.

### MODE FOR CARRYING OUT THE INVENTION

Embodiments of the present invention will now be described concretely.

The rubber additive of the present invention comprises a hydrazide compound represented by the following Formula (I): 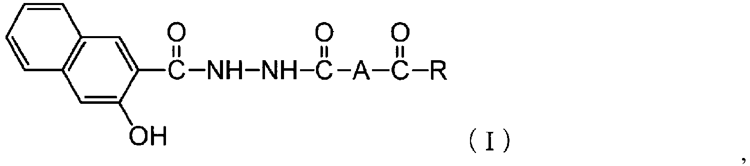 where R represents a hydroxyl group, an alkyl group having 1 to 4 carbon atoms, or an alkoxy group having 1 to 4 carbon atoms; and A represents an alkylene group having 1 to 4 carbon atoms or an alkenylene group having 2 to 4 carbon atoms.

By adding the rubber additive comprising the hydrazide compound represented by the Formula (I) to a diene-based rubber component, excellent low heat generation properties and manufacturing operability can be imparted to the resulting rubber composition.

That is, the rubber additive of the present invention can be used as a loss reducing agent; therefore, in other words, the rubber additive of the present invention encompasses a loss reducing agent comprising a hydrazide compound represented by the Formula (I), which loss reducing agent is characterized by being added to a diene-based rubber component.

Examples of the alkyl group having 1 to 4 carbon atoms include linear or branched alkyl groups having 1 to 4 carbon atoms, such as a methyl group, an ethyl group, an *n*-propyl group, an isopropyl group, an *n*-butyl group, a sec-butyl group, and a *tert*-butyl group.

Examples of the alkoxy group having 1 to 4 carbon atoms include linear or branched alkoxy groups having 1 to 4 carbon atoms, such as a methoxy group, an ethoxy group, an *n*-propoxy group, an isopropoxy group, an *n*-butoxy group, a sec-butoxy group, and a tert-butoxy group.

Examples of the alkylene group having 1 to 4 carbon atoms include linear or branched alkylene groups having 1 to 4 carbon atoms, such as a methylene group, an ethylene group, a trimethylene group, a tetramethylene group, a propylene group, and an ethyl ethylene group.

Examples of the alkenylene group having 2 to 4 carbon atoms include linear or branched alkenylene groups having 2 to 4 carbon atoms, such as a vinylene group, a 1-propenylene group, a 2-propenylene group, a 1-butenylene group, and a 2-butenylene group.

The hydrazide compound represented by the Formula (I) may include geometric isomers derived from a carbon-carbon double bond in its structural formula and, in such a case, the present invention encompasses the respective geometric isomers as well as mixtures containing them at any arbitrary ratio.

Among hydrazide compounds represented by the Formula (I), one in which R represents an alkoxy group having 1 to 4 carbon atoms and A represents an alkenylene group having 2 to 4 carbon atoms is a novel compound that has not been described in any document.

The rubber additive of the present invention is preferably a rubber additive that comprises, among hydrazide compounds represented by the Formula (I), one in which A is a methylene group, an ethylene group, or a vinylene group.

Further, the rubber additive of the present invention is preferably a rubber additive that comprises, among hydrazide compounds represented by the Formula (I), one in which R is a hydroxyl group, a methyl group, or a methoxy group.

The rubber additive of the present invention is more preferably, among hydrazide compounds represented by the Formula (I), one in which A is a vinylene group and R is a hydroxyl group or a methoxy group, still more preferably a rubber additive that comprises a compound in which A is a vinylene group and R is a hydroxyl group.

The rubber additive of the present invention is yet still more preferably a rubber additive that comprises, among hydrazide compounds represented by the Formula (I), one in which A is a methylene group and R is a methyl group.

By adding or incorporating the rubber additive of the present invention to a diene-based rubber component, superior low heat generation properties and manufacturing operability can be imparted to the resulting rubber composition.

Preferred concrete compounds of the present invention are enumerated below:

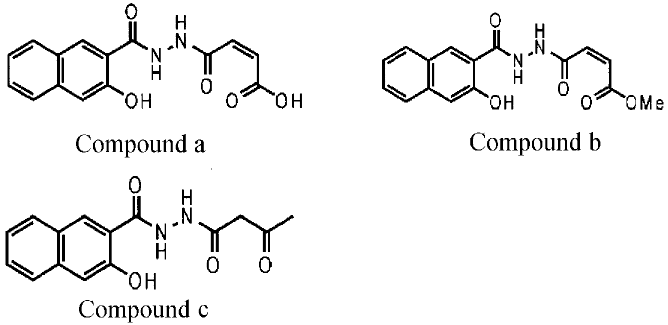

A hydrazide compound represented by the Formula (I) can be produced by, for example, a method represented by the below-described Reaction Formula-1 or 2. 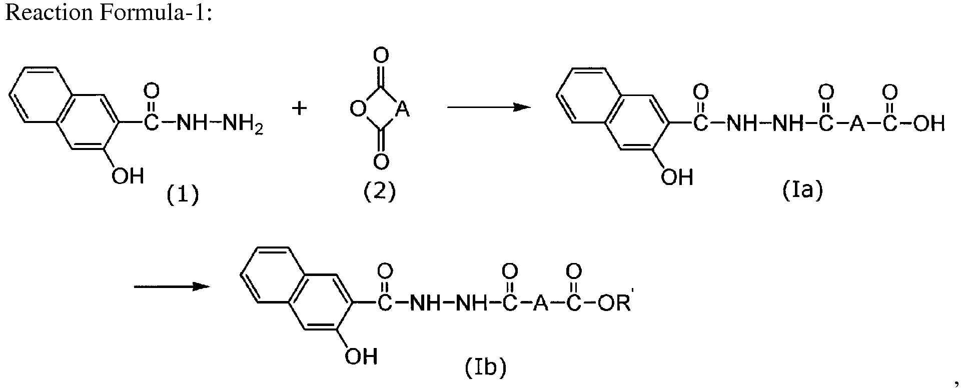 where A has the same meaning as described above; and R' represents an alkyl group having 1 to 4 carbon atoms.

According to the Reaction Formula-1, a hydrazide compound represented by the Formula (Ia) can be produced by allowing the compound represented by the Formula (1) and a cyclic acid anhydride represented by the Formula (2) to react with each other in a solvent (first step). Further, the carboxylic acid moiety of the thus obtained hydrazide compound represented by the Formula (Ia) is subjected to an ordinary esterification reaction, for example, reaction with an alcohol, whereby a hydrazide compound represented by the Formula (Ib) can be produced (second step).

### First Step

As the solvent used in this step, any known solvent can be widely used as long as it is inert to the reaction. Examples of such a solvent include ethers, such as dimethyl ether, diethyl ether, diisopropyl ether, tert-butylmethyl ether, tetrahydrofuran, dioxane, tetrahydropyran, and 1,2-dimethoxyethane; halogens, such as dichloromethane, chloroform, and carbon tetrachloride; hydrocarbons, such as hexane and cyclohexane; aromatic hydrocarbons, such as benzene, toluene, xylene, and styrene; alcohols, such as methanol, ethanol, *n*-propanol, isopropanol, *n*-butanol, and tert-butanol; nitriles, such as acetonitrile and propionitrile; amides, such as *N*-methyl-2-pyrrolidone, *N,N*-dimethylformamide, and *N,N*-dimethylacetamide; and sulfoxides, such as dimethyl sulfoxide and sulfolane. These solvents may be used individually or, as required, two or more thereof may be used in combination.

These solvents may be used in an amount of usually 1 to 100 parts by mass or so, preferably 1 to 20 parts by mass or so, with respect to 1 part by mass of the compound represented by the Formula (1).

Examples of the cyclic acid anhydride used in this step include succinic anhydride, glutaric anhydride, adipic anhydride, and maleic anhydride.

These cyclic acid anhydrides may be used in an amount of usually 1.0 to 10.0 equivalents, preferably 1.0 to 2.0 equivalents, with respect to the compound represented by the Formula (1).

The reaction of this step can be performed in a temperature range of 0°C to the boiling point of a solvent in use; however, it is usually performed at a temperature of 25 to 100°C or so, and the reaction temperature may be adjusted in accordance with the solvent(s), reaction reagent(s) and the like that are used.

The reaction time varies depending on the reaction temperature and the like and thus cannot be generalized; however, this reaction is usually completed in 0.5 to 24 hours or so.

### Second Step

In this step, an alkyl esterification reaction of a carboxylic acid that is normally performed may be applied and, for example, a method using an alkyl alcohol as an esterification agent can be employed.

As a solvent, any known solvent can be widely used as long as it is inert to the reaction. Examples of such a solvent include ethers, such as dimethyl ether, diethyl ether, diisopropyl ether, tert-butylmethyl ether, tetrahydrofuran, dioxane, tetrahydropyran, and 1,2-dimethoxyethane; halogens, such as dichloromethane, chloroform, and carbon tetrachloride; hydrocarbons, such as hexane and cyclohexane; aromatic hydrocarbons, such as benzene, toluene, xylene, and styrene; nitriles, such as acetonitrile and propionitrile; amides, such as *N*-methyl-2-pyrrolidone, *N,N*-dimethylformamide, and *N,N*-dimethylacetamide; and sulfoxides, such as dimethyl sulfoxide and sulfolane. The alcohol to be reacted, such as methanol, ethanol, *n*-propanol, isopropanol, *n*-butanol or *tert*-butanol, may also be used a solvent.

These solvents may be used in an amount of usually 1 to 100 parts by mass or so, preferably 1 to 20 parts by mass or so, with respect to 1 part by mass of the hydrazide compound represented by the Formula (Ia).

In this step, an acid is preferably used as a catalyst, and examples thereof include inorganic acids, such as hydrochloric acid, sulfuric acid, nitric acid, phosphoric acid, and boric acid; and sulfonic acids, such as methanesulfonic acid, benzenesulfonic acid, *p*-toluenesulfonic acid, and trifluoromethanesulfonic acid. These acids may be used individually or, as required, two or more thereof may be used in combination.

Such an acid catalyst may be used in an amount of usually 0.01 to 50 equivalents, preferably 0.01 to 1.0 equivalent, with respect to the hydrazide compound represented by the Formula (Ia).

The reaction of this step can be performed in a temperature range of 0°C to the boiling point of a solvent in use; however, it is usually performed at a temperature of 20 to 100°C or so, and the reaction temperature may be adjusted taking into consideration the solvent(s) and reaction reagent(s) that are used.

The reaction time varies depending on the reaction temperature and the like and thus cannot be generalized; however, this reaction is usually completed in 0.5 to 24 hours or so. 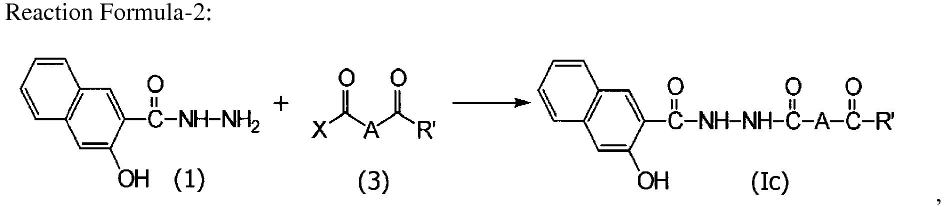 where A and R' have the same meaning as described above; and X represents a halogen atom.

According to the Reaction Formula-2, a hydrazide compound represented by the Formula (Ic) can be produced by allowing a carboxylic acid halide represented by the Formula (3) to react with the compound represented by the Formula (1).

This reaction is performed in a solvent and, as the solvent, any known solvent can be widely used as long as it is inert to the reaction. Examples of such a solvent include ether-based solvents, such as dimethyl ether, diethyl ether, diisopropyl ether, tert-butylmethyl ether, tetrahydrofuran, dioxane, tetrahydropyran, and 1,2-dimethoxyethane; aromatic solvents, such as toluene, xylene, and benzene; halogen-based solvents, such as dichloromethane and carbon tetrachloride; amide-based solvents, such as *N,N*-dimethylformamide, N,N-dimethylacetamide, and hexamethylphosphoric triamide; and sulfoxide-based solvents, such as dimethyl sulfoxide. These solvents may be used individually or, as required, two or more thereof may be used in combination.

These solvents may be used in an amount of usually 1 to 500 parts by mass or so, preferably 5 to 100 parts by mass or so, with respect to 1 part by mass of the compound represented by the Formula (1).

Examples of the carboxylic acid halide represented by the Formula (3) include halogenation products of carboxylic acids, such as 3-oxohexanoic acid, 3-oxoheptanoic acid, levulinic acid, 3-oxopentanoic acid, 4-oxohexanoic acid, 4-oxoheptanoic acid, and 5-oxohexanoic acid.

As for the amount of the compound represented by the Formula (1) to be used in this reaction, the compound represented by the Formula (1) may be used in an amount of usually 0.8 to 5.0 equivalents, preferably 1.0 to 3.0 equivalents, more preferably 1.2 to 2.0 equivalents, with respect to the carboxylic acid halide represented by the Formula (3).

This reaction is preferably performed in the presence of a base. Examples of the base to be used include aromatic amines, such as pyridine, *N,N*-dimethyl-4-aminopyridine, pyrazine, and 2,6-lutidine; tertiary aliphatic amines, such as trimethylamine, triethylamine, diisopropylethylamine, and *N*-methylpyrrolidine; and caustic compounds, such as sodium hydroxide and potassium hydroxide. These bases may be used in an amount of usually 0.8 to 5.0 equivalents, preferably 1.0 to 3.0 equivalents, more preferably 1.2 to 2.0 equivalents, with respect to the compound represented by the Formula (1).

This reaction can be performed in a temperature range of -78°C to the boiling point of a solvent in use; however, it is usually performed at a temperature of -10 to 50°C or so, preferably around room temperature.

The reaction time varies depending on the reaction temperature and the like and thus cannot be generalized; however, this reaction is usually completed in 0.5 to 24 hours or so.

In cases where A and R' of the hydrazide compound represented by the Formula (Ic) are a methylene group and a methyl group, respectively, the hydrazide compound represented by the Formula (Ic) can be produced by allowing 4-methyleneoxetan-2-one to react with the compound represented by the Formula (1).

As a solvent used in this reaction, any known solvent can be widely used as long as it is inert to the reaction. Examples of such a solvent include ethers, such as dimethyl ether, diethyl ether, diisopropyl ether, *tert*-butylmethyl ether, tetrahydrofuran, dioxane, tetrahydropyran, and 1,2-dimethoxyethane; halogens, such as dichloromethane, chloroform, and carbon tetrachloride; hydrocarbons, such as hexane and cyclohexane; aromatic hydrocarbons, such as benzene, toluene, xylene, and styrene; nitriles, such as acetonitrile and propionitrile; amides, such as *N*-methyl-2-pyrrolidone, *N,N*-dimethylformamide, and *N,N*-dimethylacetamide; and sulfoxides, such as dimethyl sulfoxide and sulfolane. These solvents may be used individually or, as required, two or more thereof may be used in combination.

These solvents may be used in an amount of usually 1 to 100 parts by mass or so, preferably 1 to 20 parts by mass or so, with respect to 1 part by mass of the compound represented by the Formula (1).

In this reaction, 4-methyleneoxetan-2-one may be used in an amount of usually 1.0 to 10 equivalents, preferably 1.0 to 2.0 equivalents, with respect to the hydrazide compound represented by the Formula (1).

When the rubber additive of the present invention is added to a diene-based rubber component, the rubber additive of the present invention is added in an amount of preferably 0.01 to 30 parts by mass, more preferably 0.1 to 10 parts by mass, with respect to 100 parts by mass of the diene-based rubber component.

The addition of the rubber additive of the present invention to the diene-based rubber component is preferably performed using a mixer, an extruder or a kneader, or by spraying.

It is noted here that the term "comprise" used herein encompasses concepts of "contain", "consist essentially of" and "consist only of".

The rubber composition of the present invention comprises, with respect to 100 parts by mass of a diene-based rubber component: 10 to 160 parts by mass of a filler; and 0.01 to 30 parts by mass of the rubber additive of the present invention.

By this constitution, the chemical interaction between the filler and the rubber component in the rubber composition is improved, so that the dispersion and the reinforcing effect of the filler are improved. Particularly, when the filler is a carbon black, the dispersion and the reinforcing effect of the carbon black are improved and, in association with this, the hysteresis loss caused by friction of the filler particles is reduced, as a result of which a low tan δ is attained. Further, the scorch performance can be improved, that is, the scorch time can be extended than ever before, while maintaining a low tan δ.

Next, the filler incorporated into the rubber composition of the present invention will be described.

In the rubber composition of the present invention, the filler is incorporated in an amount of 10 to 160 parts by mass, preferably 30 to 100 parts by mass, with respect to 100 parts by mass of the diene-based rubber component. As the filler, a carbon black is preferred; however, a carbon black and an inorganic filler may be used in combination as well. When an inorganic filler is used in combination, the inorganic filler is preferably used in an amount of 80 parts by mass or less with respect to 100 parts by mass of the diene-based rubber component. By incorporating a carbon black by itself, a stronger reinforcing effect of the carbon black can be obtained.

The carbon black as the filler used in the rubber composition of the present invention is not particularly restricted. For example, a high-, medium- or low-structure carbon black of SAF, ISAF, IISAF, N339, HAF, FEF, GPF, or SRF grade is preferably used, and a carbon black of SAF, ISAF, IISAF, N339, HAF, or FEF grade is particularly preferably used. The carbon black preferably has a nitrogen adsorption specific surface area (N₂SA, measured in accordance with JIS K 6217-2:2001) of 30 to 250 m²/g. Such a carbon black may be used individually, or two or more thereof may be used in combination.

As the inorganic filler other than the carbon black that is used in the rubber composition of the present invention is preferably silica. As this silica, a variety of commercially available silicas can be used and, thereamong, wet silica, dry silica or colloidal silica is preferably used, and a wet silica is particularly preferably used.

In cases where an inorganic filler such as silica is incorporated into the rubber composition of the present invention, it is preferred to incorporate a silane coupling agent from the standpoint of improving the reinforcing effect of the inorganic filler, and this not only imparts the rubber composition with superior manufacturing operability in rubber processing but also imparts more favorable wear resistance to the resulting pneumatic tire. The silane coupling agent used in the rubber composition of the present invention is not particularly restricted, and one which is generally and widely used can be employed.

The rubber component used in the rubber composition of the present invention is required that 50% by mass or greater thereof be constituted by a diene-based rubber, and it is preferred that 100% by mass of the rubber component be constituted by a diene-based rubber. The diene-based rubber is preferably composed of at least one selected from natural rubbers and synthetic diene-based rubbers. The synthetic diene-based rubbers may be any rubbers that contain a diene monomer as at least one portion of the monomers constituting the respective rubbers, and there is no other particular restriction. Specifically, styrene-butadiene copolymer rubbers (SBR), polybutadiene rubbers (BR), polyisoprene rubbers (IR), acrylonitrile-butadiene copolymer rubbers (NBR), butyl rubbers (IIR), halogenated butyl rubbers (e.g., chlorinated butyl rubber (CI-IIR) and brominated butyl rubber (Br-IIR)), ethylene-propylene-diene ternary copolymer rubbers (EPDM), ethylene-butadiene copolymer rubbers (EBR), propylene-butadiene copolymer rubbers (PBR) and the like can be used, and natural rubbers and synthetic diene-based rubbers may be used individually, or two or more thereof may be used as a blend. Examples of a rubber component other than the diene-based rubber include ethylene-propylene copolymer rubbers (EPM), acrylic rubbers (ACM), chlorinated polyethylenes (CM), epichlorohydrin rubbers (CO, ECO), and chlorosulfonated polyethylenes (CSM).

In the rubber composition of the present invention, as other optional components, compounding agents that are normally used in the rubber industry, examples of which include softening agents, antioxidants, silane coupling agents, vulcanization accelerators, vulcanization accelerator aids and vulcanizers, may be selected and incorporated as appropriate within a range that does not adversely affect the object of the present invention. As these additives, commercially available products can be suitably used. The rubber composition of the present invention can be produced by, for example, kneading, heating, and extruding the materials using a Banbury mixer, a roll, an intensive mixer, a biaxial extruder or the like, with an addition of such various compounding agents.

A pneumatic tire of the present invention comprises the rubber composition of the present invention, and the rubber composition of the present invention can be suitably used in at least one member of a tread portion, a side wall portion, a bead portion, an inner liner, and other reinforcing rubber part. The pneumatic tire of the present invention is not particularly restricted as long as it comprises the above-described rubber composition, and the pneumatic tire of the present invention can be produced in accordance with a conventional method. As a gas filled into the pneumatic tire, air having normal or adjusted oxygen partial pressure as well as an inert gas, such as nitrogen, argon or helium, can be used.

### EXAMPLES

The present invention will now be described in more detail by way of examples thereof.

### Production Example 1: Production of

### (Z)-4-[N'-(3-hydroxy-2-naphthoyl)diazanyl]-4-oxobut-2-enoic acid (Compound a)

After suspending 30 g of 3-hydroxy-2-naphthoic acid hydrazide in 300 mL of toluene, 16 g of maleic anhydride was added, and the resultant was stirred for 2 hours at 60°C. This reaction solution was filtered, and the thus obtained crystals were washed with toluene and thendried in vacuo, whereby 44.4 g (yield: 99%) of

### (Z)-4-[N'-(3-hydroxy-2-naphthoyl)diazanyl]-4-oxobut-2-enoic acid was obtained.

Property: yellow solid
Melting point: 241°C
¹H-NMR (300 MHz, DMSO-D₆, δppm): 6.4(s,2H), 7.3(s,1H), 7.4(t,1H), 7.5(t,1H), 7.8(d,1H), 7.9(d,1H), 8.6(s,1H), 11.0(br-s,1H), 11.4(br-s,2H), 13.3(br-s,1H) Production Example 2: Production of methyl

### (Z)-4-[N'-(3-hydroxy-2-naphthoyl)diazanyl]-4-oxobut-2-enoate (Compound b)

After suspending 10 g of (Z)-4-[*N*'-(3-hydroxy-2-naphthoyl)diazanyl]-4-oxobut-2-enoic acid produced in Production Example 1 in 150 mL of methanol, 0.44 mL of sulfuric acid was added, and the resultant was stirred under reflux for 7 hours. After further adding triethylamine thereto, the solvent was concentrated in reduced pressure, and acetone was added to the resulting residue, followed by filtration. The thus obtained solid was recrystallized using hot acetone, whereby 2.6 g (yield: 25%) of methyl (Z)-4-[*N*'-(3-hydroxy-2-naphthoyl)diazanyl]-4-oxobut-2-enoate was obtained.
Property: white solid
Melting point: 191°C
¹H-NMR (300 MHz, DMSO-D₆, δppm): 3.7(s,3H), 6.4(d,1H), 6.5(d,1H), 7.3(s,1H), 7.4(t,1H), 7.5(t,1H), 7.8(d,1H), 7.9(d,1H), 8.5(s,1H), 11.0(br-s,1H), 11.1(s,1H), 11.5(br-s,1H) Production Example 3: Production of *N*'-(3-hydroxy-2-naphthoyl)-*N*-(3-oxobutano)hydrazide

### (Compound c)

After adding 350 mL of tetrahydrofuran to 17.5 g of 4-methyleneoxetan-2-one, 35 g of 3-hydroxy-2-naphthoic acid hydrazide was added thereto in small amounts at room temperature, and 210 mL of *N,N*-dimethylformamide was subsequently further added. The resultant was stirred for 2 hours and then stirred overnight under heating at 35°C. The resulting reaction solution was filtered, and solids recovered on a filter paper was washed with tetrahydrofuran (the desired substance was dissolved), followed by addition of 460 mL of water to the filtrate. Precipitated crystals were filterd off, washed with hydrated tetrahydrofuran, and then vacuum-dried, whereby 11.6 g (yield: 23%) of *N*'-(3-hydroxy-2-naphthoyl)-*N*-(3-oxobutano)hydrazide was obtained.
Property: light brown solid
Melting point: 210°C
¹H-NMR (300 MHz, DMSO-D₆, δppm): 2.2(s,3H), 3.5(s,2H), 7.3(t,2H), 7.5 (t,1H), 7.7(d,1H), 7.9(d,1H), 8.5(s,1H), 10.7(s,1H), 10.8(br-s,1H), 11.5(br-s,1H)

### (Examples 1 to 3 and Comparative Examples 1 and 2)

Test rubber compositions were prepared in accordance with the respective formulations shown in Table 1 below. For the thus obtained rubber compositions, the tan δ, the scorch time and the Mooney viscosity were measured. The measurement methods were as follows.

### (Tan δ)

Using a viscoelasticity measuring apparatus (manufactured by Rheometric Scientific Inc.), the tan δ was measured at a temperature of 50°C, a distortion of 5% and a frequency of 15 Hz, and the thus measured tan δ was shown as an index, taking the value of Comparative Example 1 as 100. The smaller the index value, the better the low heat generation properties.

### (Scorch Time and Mooney Viscosity)

The unvulcanized rubber viscosity and the scorch time were measured in accordance with JIS K6300-1:2001 (Mooney viscosity, Mooney scorch time). The thus measured values were each shown as an index, taking the value of Comparative Example 1 as 100. As for the scorch index, the larger the index value, the better the scorch performance and the better the manufacturing operability. As for the Mooney viscosity index, the smaller the index value, the lower the viscosity and the better the manufacturing operability.

**[Table 1]**

| | | Example | | | Comparative Example | |
|---|---|---|---|---|---|---|
| | | 1 | 2 | 3 | 1 | 2 |
| | Natural rubber*¹ | 100.0 | 100.0 | 100.0 | 100.0 | 100.0 |
| | Carbon black N220*² | 50.0 | 50.0 | 50.0 | 50.0 | 50.0 |
| | Compound a | 1.0 | | | | |
| | Compound b | | 1.0 | | | |
| | Compound c | | | 1.0 | | |
| | Compound d*³ | | | | 1.0 | |
| Formulation (parts by mass) | Compound e*⁴ | | | | | 1.0 |
| | Aromatic oil*⁵ | 2.0 | 2.0 | 2.0 | 2.0 | 2.0 |
| | Stearic acid | 2.0 | 2.0 | 2.0 | 2.0 | 2.0 |
| | Antioxidant 6PPD*⁶ | 1.0 | 1.0 | 1.0 | 1.0 | 1.0 |
| | Antioxidant TMQ*⁷ | 1.0 | 1.0 | 1.0 | 1.0 | 1.0 |
| | Zinc oxide | 3.0 | 3.0 | 3.0 | 3.0 | 3.0 |
| | Vulcanization accelerator CBS*⁸ | 0.8 | 0.8 | 0.8 | 0.8 | 0.8 |
| | Sulfur | 1.5 | 1.5 | 1.5 | 1.5 | 1.5 |
| Physical property | Tan δ index | 88 | 96 | 93 | 100 | 104 |
| | Scorch index | 147 | 151 | 135 | 100 | 102 |
| | Mooney viscosity index | 102 | 100 | 103 | 100 | 103 |

| | | | | | | |
|---|---|---|---|---|---|---|
| *1: RSS#1 *2: "#80", manufactured by Asahi Carbon Co., Ltd. *3: 3-hydroxy-*N*'-(1,3-dimethylbutylidene)-2-naphthoic acid hydrazide 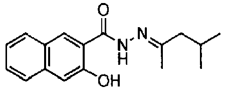 *4: the following compound described in Japanese Unexamined Patent Application Publication No. 2014-84312 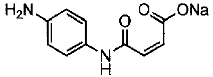 *5: "AROMAX #3", manufactured by Fuji Kosan Co., Ltd. *6: *N*-(1,3-dimethylbutyl)-*N*'-phenyl-*p*-phenylenediamine, manufactured by Ouchi Shinko Chemical Industrial Co., Ltd., trade name "NOCRAC 6C" *7: 2,2,4-trimethyl-1,2-dihydroquinoline polymer, manufactured by Ouchi Shinko Chemical Industrial Co., Ltd., trade name "NOCRAC 224" *8: *N*-cyclohexyl-2-benzothiazole sulfenamide, "SANCELER CM" manufactured by Sanshin Chemical Industry Co., Ltd. | | | | | | |

As apparent from Table 1, all of the rubber compositions of Examples 1 to 3, in which the rubber additive of the present invention was incorporated, were superior in both manufacturing operability and low heat generation properties as compared to the rubber compositions of Comparative Examples 1 and 2.

## Claims

1. A rubber additive comprising a hydrazide compound represented by the following 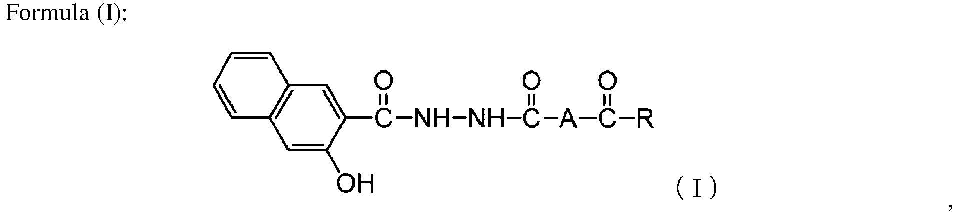 where R represents a hydroxyl group, an alkyl group having 1 to 4 carbon atoms, or an alkoxy group having 1 to 4 carbon atoms; and A represents an alkylene group having 1 to 4 carbon atoms or an alkenylene group having 2 to 4 carbon atoms.

2. The rubber additive according to claim 1, which is used as a loss reducing agent.

3. A rubber composition comprising, with respect to 100 parts by mass of a diene-based rubber component:
10 to 160 parts by mass of a filler; and
0.01 to 30 parts by mass of the rubber additive according to claim 1.

4. The rubber composition according to claim 3, wherein said diene-based rubber component contains a natural rubber.

5. The rubber composition according to claim 3, wherein said filler contains a carbon black.

6. A pneumatic tire comprising the rubber composition according to claim 3.

7. The pneumatic tire according to claim 6, wherein said rubber composition is used in at least one member of a tread portion, a side wall portion, a bead portion, an inner liner, and other reinforcing rubber part.

8. A hydrazide compound represented by the following Formula (I): 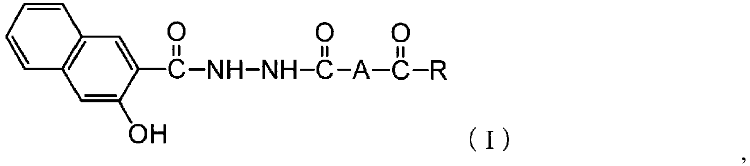 where R represents an alkoxy group having 1 to 4 carbon atoms; and A represents an alkenylene group having 2 to 4 carbon atoms.
